# FASCICULE DE BREVET EUROPEEN

(11) **EP 3 068 535 B1**
(45) Date de publication et mention de la délivrance du brevet: **10.07.2019**
(21) Numéro de dépôt: 14821696.3
(22) Date de dépôt: 13.11.2014
(51) Int. Cl.: B01L 3/00, G01N 33/49

(54) **PROCÉDÉ ET DISPOSITIF POUR TRANSFÉRER UNE PARTIE D'UN LIQUIDE CONTENU DANS UN RÉCIPIENT**
VERFAHREN UND VORRICHTUNG ZUR ÜBERTRAGUNG EINES TEILS EINER FLÜSSIGKEIT IN EINEM BEHÄLTER
METHOD AND DEVICE FOR TRANSFERRING PART OF A LIQUID HOUSED IN A CONTAINER

(30) Priorité: 14.11.2013 FR 1361112
(43) Date de publication de la demande: 21.09.2016
(73) Titulaire: Biomérieux, 69280 Marcy l'Étoile (FR)
(72) Inventeur: FOUCAULT, Frédéric, F-69280 Marcy l'Etoile (FR)
(74) Mandataire: Thibault, Jean-Marc
(86) Numéro de dépôt international: PCT/FR2014/052891
(87) Numéro de publication internationale: WO 2015/071599

(56) Documents cités:
- US-A- 5 603 695
- US-A1- 2002 177 819
- US-A1- 2004 014 203
- US-A1- 2009 082 751
- US-A1- 2009 131 864

## Description

La présente invention concerne le domaine technique du transfert d'une partie d'un liquide au sens général, contenu dans un récipient au sens général et en particulier muni d'un bouchon pénétrable par une aiguille creuse.

L'objet de l'invention trouve une application particulièrement avantageuse mais non exclusivement dans le domaine de la biologie et en particulier dans l'extraction du plasma à partir du sang contenu dans un récipient muni d'un bouchon.

Dans le domaine d'application préférée de l'invention, il est largement répandu de récupérer un échantillon de sang dans un récipient se présentant sous la forme d'un tube obturé par un bouchon tel qu'un septum pénétrable par une aiguille creuse. De nombreuses solutions ont été proposées pour obtenir du plasma à partir d'un tel échantillon de sang. Ces solutions reposent entre autres sur deux techniques possibles pour extraire du plasma à partir du sang total, à savoir la filtration et la centrifugation.

L'isolation du plasma sanguin par centrifugation nécessite la mise en oeuvre d'un appareillage complexe et onéreux pour d'une part, séparer les composants du sang en fonction de leur différence de densité en les soumettant à une force centrifuge et d'autre part, extraire le plasma de la partie du sang contenant les hématies ayant eu le temps de sédimenter. Indépendamment de la nécessité d'avoir recours à une centrifugeuse et à des tubes à centrifugation, cette technique présente un temps de centrifugation relativement long, avec une difficulté pour définir la force centrifuge pour obtenir un plasma dépourvu des éléments figurés du sang.

L'extraction du plasma par filtration nécessite l'utilisation d'un milieu de séparation pour le sang. L'article « Micro-scale blood plasma separation: from acoustophoresis to egg-beaters », publié dans 'The Royal Society of Chemistry 2013', fait une revue des diverses solutions connues pour assurer une séparation du plasma à l'échelle microscopique. L'analyse des techniques antérieures connues conduit à constater qu'il n'existe pas une solution simple, sans risque de contamination, pour extraire relativement rapidement, d'un récipient obturé contenant un prélèvement de liquide tel que du sang, une quantité non négligeable de ce liquide en vue d'être soumis à une séparation.

Le brevet US 5 603 695 décrit un dispositif permettant d'ajouter une solution tampon contenue dans une cartouche, à un anesthésique contenu dans une carpule comportant un septum pénétrable par une aiguille et un piston déplaçable par une seringue.

La cartouche est pourvue d'une aiguille supportée par une membrane et munie d'une butée. Le déplacement relatif entre la carpule et la cartouche conduit à la perforation du septum par l'aiguille qui est déplacée pour traverser la membrane et venir en contact par sa butée, sur la membrane. L'aiguille assure ainsi le transfert de la solution tampon à l'intérieur de la carpule. La cartouche est montée sur la carpule et se trouve équipée d'une paroi pourvue d'une aiguille traversant de part en part pour communiquer d'une part avec la solution tampon et d'autre part, avec une chambre délimitée entre la paroi et le septum de la carpule. Lors du déplacement relatif de la carpule, l'aiguille perce le septum de la carpule permettant le transfert de la solution tampon à l'intérieur de la carpule. Si le dispositif décrit par ce brevet permet d'ajouter une solution tampon contenue dans une cartouche, à un milieu contenu dans une carpule, un tel dispositif ne permet pas d'extraire facilement et rapidement une partie d'un liquide contenu dans un récipient.

La demande de brevet US 2002/177819 décrit une seringue d'injection à un patient, d'un médicament contenu dans un récipient muni d'un bouchon. Cette seringue comporte un support de réception du récipient muni d'une aiguille creuse adaptée pour traverser le bouchon du récipient lors de sa mise en place sur le support de réception. Cette aiguille creuse débouche dans une chambre étanche de pressurisation délimitée entre le support et un septum pénétrable par une aiguille. Le support du récipient est monté mobile en rapprochement entre l'aiguille creuse et le septum afin d'augmenter la pression à l'intérieur de la chambre et par suite du récipient par le transfert de l'air à travers l'aiguille creuse afin d'atteindre une pression de transfert avant le passage de l'aiguille creuse à travers le septum. La poursuite du déplacement du récipient conduit l'aiguille creuse à traverser le septum pour déboucher dans une chambre de distribution du médicament possédant une pression inférieure à la pression à l'intérieur du récipient permettant le transfert du contenu du récipient dans la chambre de distribution à travers l'aiguille creuse. Cette chambre de distribution est apte à communiquer avec une aiguille d'injection du médicament à un patient, lors de la poursuite du déplacement du récipient conduisant à la translation du septum et à l'écoulement du médicament à travers l'aiguille d'injection.

L'objet de l'invention vise donc à remédier aux inconvénients de l'art antérieur en proposant une nouvelle technique pour extraire simplement sans risque de contamination, et dans un temps relativement court, une partie d'un liquide contenu dans un récipient.

Un autre objet de l'invention est de proposer un procédé permettant d'extraire à plusieurs reprises, d'un même récipient, une quantité non négligeable de liquide contenu dans un tel récipient.

Pour atteindre de tels objectifs, l'invention concerne un procédé pour transférer une partie d'un liquide contenu dans un récipient muni d'un bouchon. Selon l'invention, ce procédé est conforme à la revendication 1.

De plus, le procédé selon l'invention peut comporter en outre en combinaison, au moins l'une et/ou l'autre des caractéristiques additionnelles suivantes :
- pour un récipient contenant du sang, assurer l'extraction du plasma en tant que partie du liquide ;
- après l'extraction du plasma, récupérer le système filtrant en vue d'analyses ;
- le fluide de la chambre de pressurisation comporte un milieu de traitement pour le liquide contenu dans le récipient ;
- pour assurer l'extraction du plasma, à atteindre une pression de transfert comprise entre la pression atmosphérique et la pression atmosphérique plus 3 bars ;
- au terme de l'écoulement du fluide dans la chambre de distribution, assurer l'écartement relatif entre l'aiguille creuse et le septum pénétrable par aiguille, de manière que l'aiguille creuse communique avec l'intérieur de la chambre de pressurisation afin d'assurer, par le fluide sous pression de ladite chambre, le retour, à l'intérieur du récipient, du liquide contenu dans l'aiguille ;
- à régler la pression de la chambre de pressurisation en assurant la mise à la pression atmosphérique de la chambre de pressurisation tant que l'écartement entre l'aiguille creuse et le septum pénétrable par aiguille, n'atteint pas une valeur déterminée ;
- mettre en communication l'intérieur du récipient et la chambre de pressurisation, en amenant le récipient afin que l'aiguille creuse portée par la chambre de pressurisation et débouchant dans cette dernière, traverse le bouchon du récipient ;
- lors d'une phase d'étalonnage préalable à la mise en communication du récipient avec la chambre de pressurisation, placer le septum et l'aiguille creuse en position de proximité et à injecter dans la chambre à travers l'aiguille creuse, un fluide de pressurisation.

Un autre objet de l'invention est de proposer un dispositif simple, pour extraire sans risque de contamination, et dans un temps très court, une partie d'un liquide contenu dans un récipient.

Pour atteindre un tel objectif, le dispositif, selon l'invention, pour transférer une partie d'un liquide contenu dans un récipient muni d'un bouchon est conforme à la revendication 12.

Diverses autres caractéristiques ressortent de la description faite ci-dessous en référence aux dessins annexés qui montrent, à titre d'exemples non limitatifs, des formes de réalisation de l'objet de l'invention.
La **Figure 1** est une vue en perspective montrant un exemple de réalisation du dispositif conforme à l'invention d'extraction d'un liquide contenu dans un récipient.
La **Figure 2** est une vue en coupe élévation montrant le dispositif conforme à l'invention dans une position initiale d'extraction.
La **Figure 3** est une vue en coupe élévation montrant le dispositif conforme à l'invention dans une position intermédiaire d'extraction.
La **Figure 4** est une vue à plus grande échelle, en coupe élévation montrant le dispositif conforme à l'invention dans une position d'extraction d'une partie du liquide contenue dans un récipient.
La **Figure 5** est une vue en perspective montrant le montage d'un récipient sur le piston du dispositif d'extraction conforme à l'invention.
La **Figure 6** est une vue en coupe du corps de réception du piston du dispositif d'extraction conforme à l'invention.

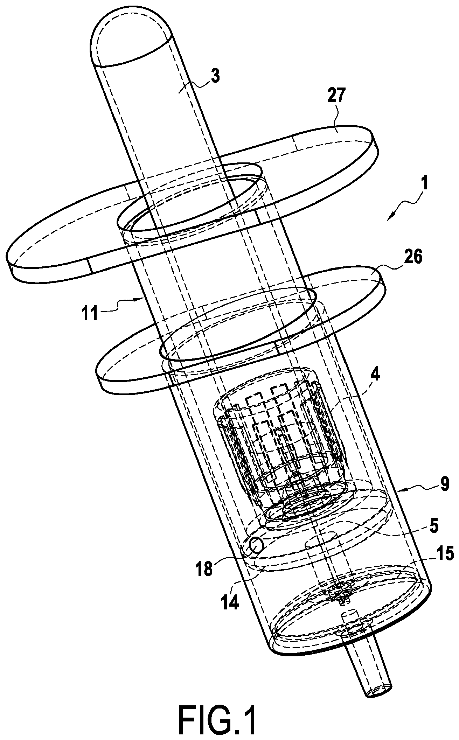

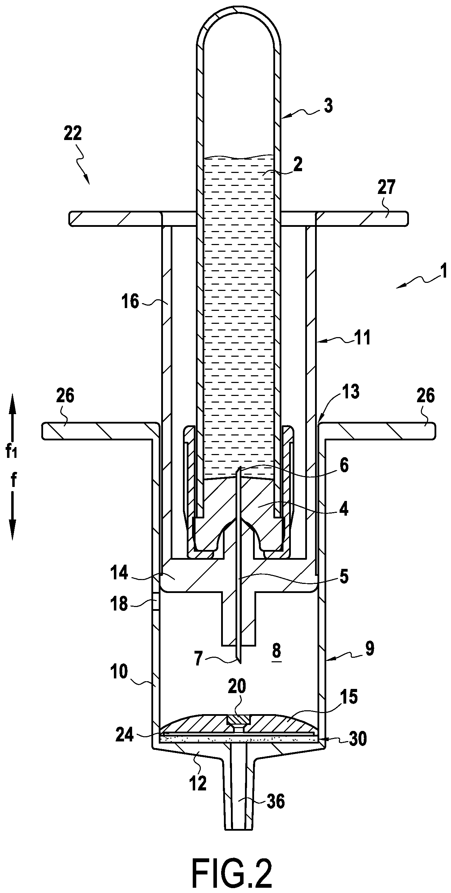

Tel que cela ressort plus précisément des **Fig. 1** et **2**, l'objet de l'invention concerne un dispositif **1** pour transférer une partie d'un liquide **2** contenu dans un récipient **3** se présentant classiquement sous la forme d'un tube fermé hermétiquement par un bouchon **4.** Selon une application préférée, le récipient **3** est un tube à prélèvement de sang sous vide mais il est clair que le dispositif selon l'invention est apte à extraire une partie du contenu d'un récipient se présentant sous une forme différente d'un tube à prélèvement.

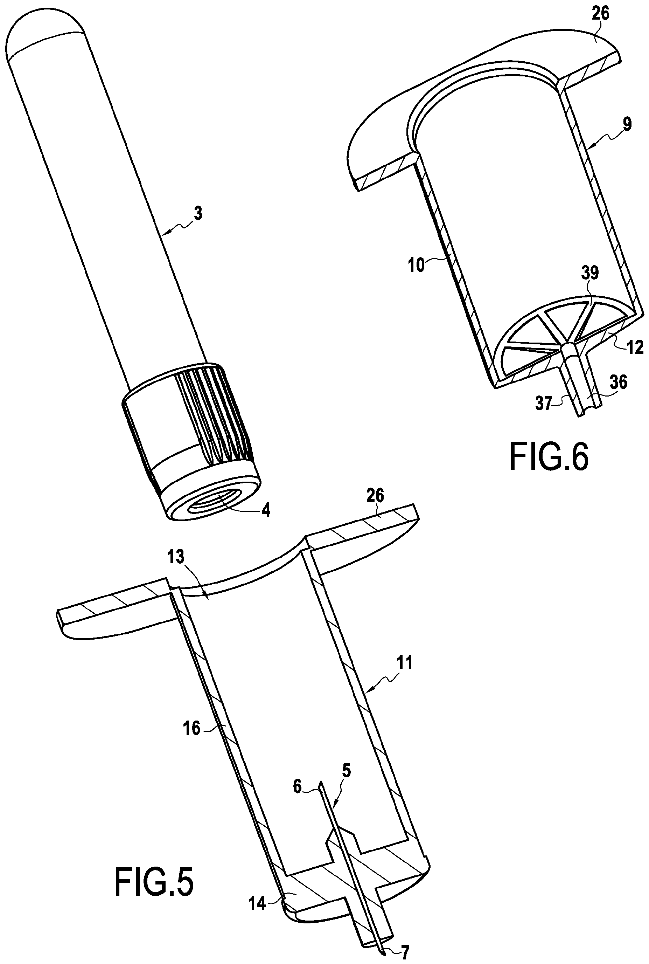

Selon une caractéristique de l'invention, le récipient **3** est apte à être traversée par une aiguille creuse **5** à travers laquelle est transférée ou extraite une partie du liquide contenu à l'intérieur du récipient. Selon l'application préférée à un tube de prélèvement de sang, le bouchon **4** tel qu'un septum est pénétrable par l'aiguille creuse **5.** Tel que cela ressort plus précisément des **Fig. 2** et **5****,** l'aiguille creuse **5** est pourvue d'une extrémité distale **6** de pénétration à travers le bouchon **4** du récipient et d'une extrémité proximale **7** dont la fonction apparaîtra plus précisément dans la suite de la description. Dans l'exemple illustré à la **Fig. 5**, le bouchon **4** du récipient **3** est traversé par l'extrémité distale **6** de l'aiguille creuse **5** à la suite d'un rapprochement relatif entre le récipient **3** et l'aiguille creuse **5**. Bien entendu, il peut être prévu de réaliser un bouchon **4** équipé dès l'origine de l'aiguille creuse **5.**

Conformément à l'invention, le dispositif **1** comporte une chambre étanche de pressurisation **8** d'un fluide contenu dans une telle chambre. Cette chambre de pressurisation **8** est délimitée entre un corps **9** et un piston **11** coopérant de manière étanche avec le corps **9.** Tel que cela ressort des **Fig. 1** et **2****,** le corps **9** se présente sous la forme d'un tube **10** de section droite transversale circulaire, muni à une extrémité, d'un fond **12** et à son extrémité opposée, d'une ouverture **13** pour le passage du piston **11.** Le piston **11** comporte une cloison rigide **14** pourvue ou non d'un joint et présentant une forme complémentaire avec le tube **10** du corps pour coopérer ensemble de manière étanche. Le tube **10** est pourvu au niveau de son fond **12,** d'une cloison de fermeture **15** permettant de délimiter avec la cloison rigide **14** du piston et la paroi du tube **10** située entre ces cloisons, la chambre étanche de pressurisation **8** à volume variable.

Selon une caractéristique préférée de réalisation, le piston **11** est pourvu de l'aiguille creuse **5** dont l'extrémité distale **6** est tournée en direction opposée de la chambre de pressurisation **8** en s'étendant en saillie à partir d'un côté de la cloison rigide **14**. Selon une caractéristique avantageuse de réalisation, la longueur de l'extrémité distale **6** de l'aiguille creuse **5** qui s'étend en saillie à partir de la cloison rigide **14** est suffisante pour traverser le bouchon **4** et déboucher à l'intérieur du récipient **3**. L'extrémité proximale **7** de l'aiguille creuse **5** s'étend en direction de la chambre de pressurisation **8** et en saillie à partir de l'autre côté de la cloison rigide **14.** Ainsi, l'aiguille creuse **5** est fixée de manière étanche par tous moyens appropriés à cette cloison rigide **14** en la traversant de part en part et en dépassant de part et d'autre de cette cloison.

Selon une autre caractéristique préférée de réalisation, le piston **11** est pourvu d'un manchon de protection **16** entourant à distance, l'extrémité distale **6** de l'aiguille creuse **5**, pour délimiter intérieurement, un volume de réception pour au moins une partie du récipient **3.** Avantageusement, le manchon de protection **16** s'élève à partir de la cloison rigide **14**, sur une hauteur supérieure à la longueur de l'extrémité distale **6** de l'aiguille creuse **5** de manière à éviter toute blessure par cette partie de l'aiguille creuse.

Il ressort de la description qui précède que le piston **11** et le corps **9** sont montés mobiles en translation relativement entre eux selon un sens de rapprochement représenté par la flèche **f** et un sens d'écartement de sens opposé, représenté par la flèche **f1**. Ainsi, le déplacement du piston **11** selon le sens de rapprochement conduit à diminuer le volume de la chambre **8** et par suite, à augmenter la pression du fluide à l'intérieur de la chambre **8**.

Il est à noter que lors de l'utilisation du dispositif **1** dans laquelle le bouchon **4** du récipient **3** est traversé par l'extrémité distale **6** de l'aiguille creuse **5,** cette chambre de pressurisation **8** communique grâce à l'aiguille creuse **5**, avec l'intérieur du récipient **3** dont la pression présente une valeur identique à celle de la chambre **8.** Aussi, l'augmentation de la pression du fluide à l'intérieur de la chambre **8** entraine une augmentation correspondante de la pression du fluide à l'intérieur du récipient **3**.

Selon une caractéristique avantageuse de réalisation, le dispositif **1** selon l'invention comporte un système **18** de réglage de la pression de la chambre de pressurisation **8** et par suite, du récipient **3**. Dans l'exemple illustré, le système **18** de réglage de la pression de la chambre de pressurisation **8** est réalisé par l'intermédiaire d'un trou traversant aménagé dans le tube **10** pour mettre à la pression atmosphérique la chambre **8** tant que le piston **11** occupe une position en retrait du trou **18.** Ainsi, dès que la position du piston **11** est telle que la chambre ne communique plus avec le trou **18,** alors la compression du fluide peut intervenir. Ce système **18** permet de régler la pression de la chambre de pressurisation **8** en assurant sa mise à la pression atmosphérique tant que l'écartement entre l'aiguille creuse **5** et d'un septum **20**, n'atteint pas une valeur déterminée par la position du trou **18**.

Avantageusement, le système **18** permet de régler à une valeur variable, la valeur de la pression de la chambre de pressurisation **8**. Selon un exemple de réalisation, le système **18** comporte un coulisseau monté mobile dans le sens de déplacement du piston et muni d'un trou adapté pour communiquer avec une fente aménagée dans le tube **10** et fermée par le coulisseau à l'exception du trou. La translation du coulisseau permet de régler le positionnement du trou de communication à la pression atmosphérique, et par suite de régler le volume de la chambre **8.** Selon un autre exemple de réalisation, la cloison de fermeture **15** du corps **9** est montée mobile par rapport au tube **10** (par exemple par vissage) pour permettre de faire varier le volume de la chambre **8.**

Selon une caractéristique de l'invention, la chambre de pressurisation **8** est équipée d'un septum **20** positionné pour être pénétrable par l'extrémité proximale **7** de l'aiguille creuse **5** après déplacement de cette dernière selon la direction de rapprochement. A cet effet, la cloison de fermeture **15** du corps **9** est équipé du septum **20** qui se trouve ainsi positionné en face de l'extrémité proximale **7** de l'aiguille creuse **5**. Par exemple, le septum **20** peut être réalisé entre autres par bi-injection, surmoulage ou insertion mécanique après moulage.

Avantageusement, le dispositif **1** selon l'invention comporte un système de guidage en translation de l'extrémité proximale **7** de l'aiguille creuse **5**, en amont du septum **20**. Ce système de guidage non représenté est adapté pour positionner l'aiguille creuse afin que l'extrémité proximale **7** traverse correctement le septum **20**.

Le dispositif **1** selon l'invention comporte un système de rapprochement relatif **22** entre l'aiguille creuse **5** et le septum **20** pour assurer selon une première course, une augmentation de la pression du fluide à l'intérieur de la chambre **8** jusqu'à atteindre une pression de transfert, et dans une deuxième course subséquente à la première, la traversée du septum **20** par l'aiguille creuse **5** afin que l'aiguille creuse **5** débouche dans une chambre de distribution **24** du liquide possédant une pression inférieure à la pression de transfert. Cette chambre de distribution **24** est ainsi séparée de la chambre de pressurisation **8** notamment par l'intermédiaire du septum **20** qui est en contact d'un côté avec la chambre de distribution **24** et du côté opposé avec la chambre de pressurisation **8**.

Le système **22** qui est adapté pour rapprocher notamment l'aiguille creuse **5** et le septum **20** exerce un effort de poussée mécanique sur l'aiguille creuse selon le sens **f** et/ou le septum **20** selon le sens **f1**. Selon la variante de réalisation illustrée sur les dessins, le système de rapprochement **22** est du type manuel mais il clair que le déplacement entre l'aiguille creuse **5** et le septum **20** peut être motorisé.

Pour favoriser l'application manuelle d'un effort de poussée, le tube **10** du corps **9** comporte extérieurement, au niveau de son ouverture **13,** deux ailettes **26** de préhension tandis que l'embout de protection **16** est muni, à l'opposé de la cloison rigide **14,** d'une collerette d'appui **27.** Classiquement, un effort de poussée est exercé sur la collerette **27** du manchon du piston **11** tandis que le corps **9** est maintenu en position à l'aide des ailettes **26**. Selon cet exemple de réalisation, un système de visualisation de l'enfoncement du piston **11** dans le corps **9** est prévu pour maîtriser la pression manuelle appliquée. Par exemple, des graduations sont aménagées sur le piston **11** pour visualiser l'enfoncement du piston **11**.

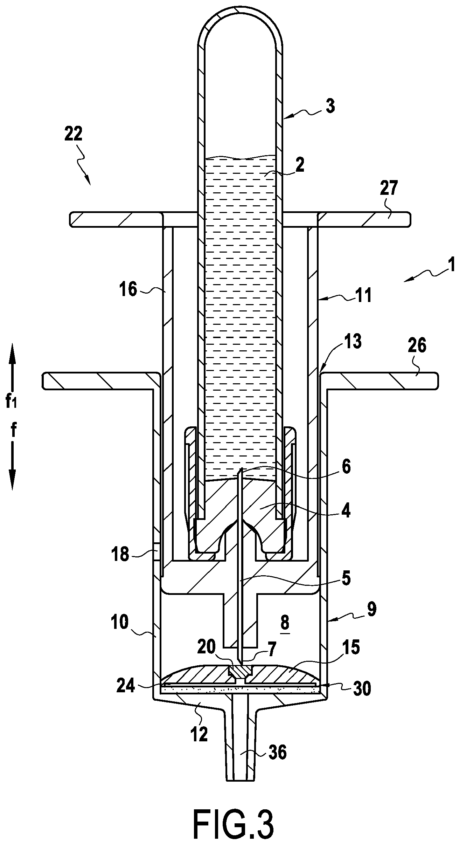

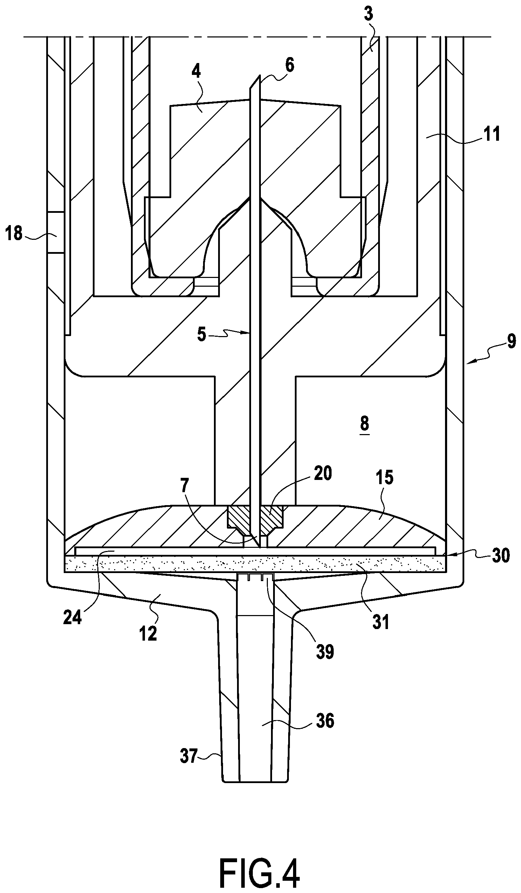

Il ressort de la description qui précède que le piston **11** est apte à occuper :
- une première position illustrée à la **Fig.** 2 dans laquelle la chambre **8** et par suite le récipient **3**, sont à la pression atmosphérique par le trou **18** ;
- une deuxième position illustrée à la **Fig. 1** permettant d'augmenter la pression à l'intérieur de la chambre **8** et par suite du récipient **3** lors du rapprochement entre les cloisons **14** et **15** ;
- une troisième position illustrée à la **Fig. 3** pour laquelle l'aiguille creuse **5** est positionnée juste avant de traverser le septum **20** ;
- et une quatrième position illustrée à la **Fig. 4** pour laquelle l'aiguille creuse **5** traverse le septum **20** et communique avec la chambre de distribution **24** qui est avantageusement à la pression atmosphérique.

Selon une caractéristique avantageuse de réalisation, le dispositif **1** selon l'invention comporte une butée de limitation du mouvement de rapprochement des cloisons **14** et **15**, dans une position prédéterminée dans laquelle l'aiguille creuse **5** traverse, par son extrémité proximale **7**, le septum **20** pour déboucher dans la chambre de distribution **24** (**Fig. 4****).** Dans l'exemple illustré **Fig. 4**, l'extrémité inférieure de la cloison **14** vient directement en butée sur la cloison **15**. Un autre système de butée non représentée pourra être réalisé de toute autre manière appropriée, par exemple par aménagement sur le manchon **16** pour venir en appui sur les oreilles du corps **10**.

Selon une caractéristique avantageuse de réalisation qui sera mieux comprise dans la suite de la description, le système de rapprochement **22** assure également un écartement relatif entre l'aiguille creuse **5** et le septum **20** pénétrable par l'aiguille. Ce système **22** qui est adapté pour écarter l'aiguille creuse **5** du septum **20** exerce un effort de poussée mécanique sur l'aiguille creuse **5** selon le sens **f1** et/ou le septum **20** selon le sens **f.** Selon la variante de réalisation illustrée sur les dessins, le système d'écartement **22** est du type manuel mais il clair que le déplacement entre l'aiguille creuse **5** et le septum **20** peut être motorisé.

La chambre de distribution **24** est pourvue d'un système filtrant **30** pour assurer l'extraction d'une phase liquide à partir du liquide transféré dans ladite chambre. Selon une application préférée de l'invention, le système filtrant **30** est adapté à extraire du plasma en tant que partie de liquide extraite du récipient **3** contenant du sang.

Tel que cela ressort plus précisément de la **Fig. 4**, le système filtrant **30** comporte un milieu filtrant **31** maintenu en position sur une structure de support telle que le fond **12** du corps **9.** Avantageusement, le milieu filtrant **31** est monté à l'intérieur de la chambre de distribution **24** pour s'étendre à distance du septum et en particulier de la cloison de fermeture **15.** Ainsi, la chambre de distribution **24** présente entre la cloison de fermeture **15** et la surface supérieure du milieu filtrant **31,** un volume de récupération du liquide sortant de l'extrémité proximale **7** de l'aiguille creuse **5.** Cette chambre de distribution **24** permet d'assurer la répartition du liquide sur toute la surface du milieu filtrant **31.**

Avantageusement, le fond **12** du corps **9** sur lequel repose le milieu filtrant **31** est incurvé en direction d'un orifice **36** de sortie pour le liquide filtré, aménagé dans un embout **37** s'étendant en saillie à partir du fond **12.** De préférence, le fond **12** sur lequel repose le milieu filtrant **31** est pourvu de stries ou de nervures **39** sur le fond **12** évitant ainsi le colmatage du milieu filtrant **31** et limitant la perte de charge **(****Fig. 6****).**

Dans l'exemple illustré sur les dessins, la chambre de distribution **24** est délimitée par une partie du corps **9** de réception du piston. Il est à noter qu'il peut être envisagé de réaliser cette chambre **24** par un boitier muni du système filtrant et rapporté sur le corps **9.** Un montage amovible de la chambre de distribution **24** peut être prévu, notamment pour récupérer facilement le milieu filtrant, si celui-ci doit être soumis à des analyses ultérieures. Le plus souvent, le dispositif complet se présentera sous la forme d'un consommable jetable, sans qu'une quelconque manipulation du milieu filtrant **31** ne soit opérée, de sorte qu'un tel montage amovible ne sera pas prévu.

La mise en oeuvre du dispositif **1** conforme à l'invention pour transférer une partie d'un liquide contenu dans un récipient **3** découle directement de la description qui précède.

La méthode de transfert consiste à mettre en communication, à l'aide de l'aiguille creuse **5,** l'intérieur du récipient **3** et la chambre de pressurisation **8.** A cet effet, le récipient **3** est introduit à l'intérieur du manchon **16** jusqu'à ce que l'extrémité distale **6** de l'aiguille creuse **5** traverse le bouchon **4.** Si la chambre de pressurisation **8** est à la pression atmosphérique grâce au trou **18**, alors le récipient **3** est également placé à la pression atmosphérique **(****Fig. 2****).**

Le procédé consiste ensuite à rapprocher relativement l'aiguille creuse **5** et le septum **20** pour augmenter la pression à l'intérieur de la chambre **8** et par suite du récipient **3.** L'application d'un effort de poussée sur le piston **11** conduit à son déplacement selon le sens **f**, jusqu'à une position au-delà du trou **18** dans laquelle la chambre **8** et le récipient **3** forment ensemble une enceinte hermétique. La poursuite du déplacement du piston **11** selon le sens **f** permet d'augmenter la pression du fluide dont une partie est transférée dans le récipient **3** à travers l'aiguille creuse **5.** Le déplacement du piston **11** est réalisé selon une course déterminée de manière que le piston **11** se trouve positionné juste avant le passage de l'aiguille creuse **5** à travers le septum **20**. Dans cette position illustrée à la **Fig. 3**, la pression à l'intérieur de la chambre **8** et du récipient **3** atteint une valeur de pression souhaitée appelée pression de transfert. Typiquement, dans le cas où le liquide contenu dans le récipient **3** est du sang duquel une extraction de plasma est souhaitée, la pression de transfert est comprise entre la pression atmosphérique (notamment la pression atmosphérique standard exercée au niveau moyen de la mer qui est de 1,013.10⁵ Pa) et la pression atmosphérique plus 3 bars.

Le procédé consiste ensuite à poursuivre le rapprochement relatif entre l'aiguille creuse **5** et le septum **20** afin que l'extrémité proximale **7** de l'aiguille creuse **5** traverse le septum **20** pour déboucher dans la chambre de distribution **24 (****Fig. 4****).** Dans la mesure où la pression de cette chambre de distribution **24** présente une pression (typiquement atmosphérique) inférieure à la pression de transfert de la chambre **8** et du récipient **3,** une partie du liquide du récipient **3** est transférée, sous l'effet de cette différence de pression, dans ladite chambre de distribution **24.** Le liquide provenant du récipient **3** passe ainsi à travers l'aiguille creuse **5** et s'écoule à partir de l'extrémité proximale **7,** dans la chambre de distribution **24,** jusqu'à l'équilibre des pressions entre le récipient **3** et la chambre de distribution **24.**

La chambre de distribution **24** est équipée du système filtrant pour assurer l'extraction du plasma à partir du sang récupéré dans la chambre **24,** alors le plasma s'écoule à partir de l'orifice de sortie **36.**

Selon une variante avantageuse de ce procédé d'extraction, cette méthode consiste au terme de l'écoulement du fluide dans la chambre de distribution **24,** à assurer l'écartement relatif entre l'aiguille creuse **5** et le septum **20** de manière à retirer du septum **20,** l'aiguille creuse **5** qui vient communiquer par son extrémité proximale **7,** avec l'intérieur de la chambre de pressurisation **8.** Dans la mesure où le fluide à l'intérieur de la chambre **8** présente une pression supérieure à celle du récipient **3,** une partie de ce fluide passe à travers l'aiguille creuse **5** entrainant le retour du liquide contenu dans l'aiguille **5,** à l'intérieur du récipient **3.** Ainsi, le liquide resté éventuellement à l'intérieur de l'aiguille ne peut s'écouler à partir de cette aiguille.

Il ressort de la description qui précède que le dispositif **1** selon l'invention permet d'extraire en toute sécurité, une partie d'un liquide contenu dans un récipient **3.** Cette extraction est réalisée de manière simple et peu onéreuse puisqu'elle procède uniquement d'une poussée mécanique. Le dispositif **1** est de préférence à usage unique pour chaque récipient. Il est à noter que le système filtrant peut être récupéré pour analyses.

Avantageusement, ce dispositif permet d'extraire directement du plasma à partir d'un tube de prélèvement de sang. Des essais réalisés avec un milieu filtrant de section circulaire et de 20 mm de diamètre ont montré que le dispositif **1** selon l'invention permet d'extraire au moins 200 µl de plasma en moins de 3 min. Par ailleurs, il a été possible d'extraire 3 fois de suite 200 µl de plasma à partir d'un même tube de prélèvement de sang de 4 ml, en changeant à chaque fois le matériau de filtration.

Il est à noter que le dispositif **1** selon l'invention offre l'avantage de pouvoir adapter, par le système **22,** la pression de la chambre de pressurisation **8** en fonction du volume de fluide contenu à l'intérieur du récipient **3,** ce volume de fluide pouvant varier à la suite d'extractions successives ou en fonction des niveaux de remplissage des récipients **3.** Cette régulation peut être réalisée visuellement ou de manière automatique.

Selon un mode particulier de réalisation, le fluide de la chambre de pressurisation **8** est de l'air. Bien entendu, le fluide de la chambre **8** peut être de nature différente tel qu'un liquide non miscible avec le liquide contenu dans le récipient **3**. Dans le même sens, le fluide de la chambre de pressurisation **8** peut comporter un milieu de traitement pour le liquide contenu dans le récipient **3,** tout en contenant une quantité de gaz suffisante pour autoriser la compression/pressurisation.

Selon une variante de mise en oeuvre du dispositif **1,** une phase d'étalonnage peut être réalisée pour contrôler la pressurisation initiale de la chambre de pressurisation **8**. Préalablement à la mise en communication du récipient **3** avec la chambre de pressurisation **8,** cette phase vise à placer le septum **20** et l'aiguille creuse **5** en position de proximité c'est à dire l'aiguille creuse juste avant de traverser le septum **20**. Un fluide de pressurisation est injecté dans la chambre **8** à travers l'aiguille creuse **5** et à partir de son extrémité distale **6** conduisant à positionner le piston mobile **11** à une distance déterminée de la cloison de fermeture **15** pour délimiter une chambre avec un volume de fluide souhaitée.

Si le dispositif **1** selon l'invention est particulièrement adapté pour extraire du plasma d'un échantillon de sang, le dispositif **1** selon l'invention permet d'extraire une partie de tout liquide biologique contenu dans un récipient **3,** comme par exemple de l'urine.

L'invention n'est pas limitée aux exemples décrits et représentés car diverses modifications peuvent y être apportées sans sortir du cadre dûment défini par les revendications.

## Revendications

1. Procédé pour transférer une partie d'un liquide contenu dans un récipient **(3)** muni d'un bouchon **(4),** consistant :
- à mettre en communication, à l'aide d'une aiguille creuse **(5)** traversant le bouchon **(4)** du récipient, l'intérieur du récipient et une chambre étanche de pressurisation **(8)** d'un fluide, équipée d'un septum (**20**) pénétrable par aiguille et présentant un volume variable à la suite du déplacement relatif entre l'aiguille creuse **(5)** et le septum (**20**) pénétrable par aiguille ;
- à assurer le rapprochement relatif entre l'aiguille creuse **(5)** et le septum (**20**) pénétrable par aiguille selon une course déterminée afin d'augmenter la pression à l'intérieur de la chambre **(8)** et par suite du récipient par le transfert du fluide à travers l'aiguille creuse et d'atteindre une pression de transfert juste avant le passage de l'aiguille creuse à travers le septum (**20**) ;
- à poursuivre le rapprochement relatif entre l'aiguille creuse **(5)** et le septum (**20**) pénétrable par aiguille afin que l'aiguille creuse traverse le septum pour déboucher dans une chambre (**24**) de distribution du liquide possédant une pression inférieure à la pression de transfert afin d'assurer, sous l'effet de cette différence de pression, le transfert à travers l'aiguille creuse (**5**), d'une partie du liquide du récipient **(3)** dans ladite chambre de distribution (**24**),
**caractérisé en ce qu'**il consiste à assurer l'écoulement du liquide transféré dans la chambre de distribution **(24),** à travers un système filtrant (**30**) pour assurer l'extraction d'une partie du liquide.

2. Procédé selon la revendication 1, **caractérisé en ce qu'**il consiste, pour un récipient **(3)** contenant du sang, à assurer l'extraction du plasma en tant que partie du liquide.

3. Procédé selon la revendication 2, **caractérisé en ce qu'**il consiste après l'extraction du plasma, à récupérer le système filtrant (**30**) en vue d'analyses.

4. Procédé selon l'une des revendications 1 à 3, **caractérisé en ce que** le fluide de la chambre de pressurisation **(8)** comporte un milieu de traitement pour le liquide contenu dans le récipient **(3)** et une quantité de gaz suffisante pour autoriser la compression / pressurisation.

5. Procédé selon la revendication 2, **caractérisé en ce qu'**il consiste, pour assurer l'extraction du plasma, à atteindre une pression de transfert comprise entre la pression atmosphérique et la pression atmosphérique plus 3 bars.

6. Procédé selon l'une des revendications 1 à 5, **caractérisé en ce qu'**il consiste au terme de l'écoulement du fluide dans la chambre de distribution **(24),** à assurer l'écartement relatif entre l'aiguille creuse **(5)** et le septum (**20**) pénétrable par aiguille, de manière que l'aiguille creuse **(5)** communique avec l'intérieur de la chambre de pressurisation **(8)** afin d'assurer, par le fluide sous pression de ladite chambre, le retour, à l'intérieur du récipient **(3),** du liquide contenu dans l'aiguille (**5**).

7. Procédé selon l'une des revendications 1 à 6, **caractérisé en ce qu'**il consiste à adapter la pression de la chambre de pressurisation **(8)** en fonction du volume de fluide contenu à l'intérieur du récipient **(3).**

8. Procédé selon la revendication 7, **caractérisé en ce qu'**il consiste à régler la pression de la chambre de pressurisation **(8)** en assurant la mise à la pression atmosphérique de la chambre de pressurisation tant que l'écartement entre l'aiguille creuse **(5)** et le septum (**20**) pénétrable par aiguille, n'atteint pas une valeur déterminée.

9. Procédé selon l'une des revendications 1 à 8, **caractérisé en ce qu'**il consiste à mettre en communication l'intérieur du récipient **(3)** et la chambre de pressurisation **(8),** en amenant le récipient afin que l'aiguille creuse **(5)** portée par la chambre de pressurisation et débouchant dans cette dernière, traverse le bouchon **(4)** du récipient.

10. Procédé selon l'une des revendications 1 à 9, **caractérisé en ce qu'**il consiste lors d'une phase d'étalonnage préalable à la mise en communication du récipient **(3)** avec la chambre de pressurisation **(8),** à placer le septum (**20**) et l'aiguille creuse **(5)** en position de proximité et à injecter dans la chambre **(8)** à travers l'aiguille creuse **(5),** un fluide de pressurisation.

11. Procédé selon la revendication 1, **caractérisé en ce que** le système de rapprochement relatif **(22)** est commandé au terme de l'écoulement du fluide dans la chambre de distribution **(24),** pour assurer un écartement relatif entre l'aiguille creuse **(5)** et le septum (**20**) pénétrable par aiguille, de manière à retirer du septum (**20**), l'aiguille creuse afin que cette dernière communique avec l'intérieur de la chambre de pressurisation **(8)** afin d'assurer, par le fluide sous pression, le retour du liquide contenu dans l'aiguille **(5)** à l'intérieur du récipient (**3**).

12. Dispositif pour transférer une partie d'un liquide contenu dans un récipient **(3)** muni d'un bouchon **(4), caractérisé en ce qu'**il comprend :
- une aiguille creuse **(5)** pourvue d'une extrémité distale **(6)** de pénétration à travers le bouchon du récipient et d'une extrémité proximale **(7)** ;
- une chambre **(8)** de pressurisation d'un fluide traversée par l'extrémité proximale **(7)** de l'aiguille creuse de transfert et équipée d'un septum (**20**) positionné pour être pénétrable par l'extrémité proximale **(7)** de l'aiguille après déplacement de cette dernière, cette chambre **(8)** étant étanche et séparée par l'intermédiaire du septum (**20**) d'une chambre **(24)** de distribution du liquide dans laquelle peut déboucher l'extrémité proximale de l'aiguille creuse **(5),** et
- un système **(22)** de rapprochement relatif entre l'aiguille creuse **(5)** et le septum (**20**) pour assurer une augmentation de la pression du fluide à la suite dudit rapprochement, ce système de rapprochement étant commandé selon une course déterminée pour dans une première phase, augmenter la pression à l'intérieur de la chambre étanche **(8)** jusqu'à atteindre une pression de transfert juste avant le passage de l'aiguille creuse **(5)** à travers le septum (**20**) et dans une deuxième phase, que l'aiguille creuse **(5)** traverse le septum (**20**) pour déboucher dans la chambre **(24)** de distribution du liquide possédant une pression inférieure à la pression de transfert afin d'assurer, sous l'effet de cette différence de pression, le transfert d'une partie du liquide du récipient **(3)** dans ladite chambre de distribution (**24**), la chambre de distribution **(24)** comportant un système filtrant (**30**) pour assurer l'extraction d'une phase liquide à partir du liquide transféré dans ladite chambre.

13. Dispositif selon la revendication 12, **caractérisé en ce que** le système filtrant (**30**) comporte un milieu filtrant (**31**) s'étendant à distance du septum pénétrable par l'aiguille pour assurer la répartition du liquide sur le milieu filtrant (**31**).

14. Dispositif selon la revendication 13, **caractérisé en ce que** le milieu filtrant **(31)** est maintenu en position sur une structure de support sur laquelle repose le milieu filtrant.

15. Dispositif selon l'une des revendications 12 à 14, **caractérisé en ce que** le système de rapprochement **(22)** comporte un corps de réception **(9)** pour un piston **(11)** délimitant entre eux la chambre de pressurisation **(8),** le corps de réception **(9)** étant équipé du septum (**20**) et le piston (**11**) et le corps étant mobile en translation relativement entre eux sous l'action d'un effort de poussée mécanique exercé soit sur l'aiguille creuse **(5)** selon le sens f et/ou le septum (**20**) selon le sens f1, soit sur l'aiguille creuse **(5)** selon le sens f1 et/ou le septum (**20**) selon le sens f.

16. Dispositif selon la revendication 15, **caractérisé en ce que** le piston **(11)** est pourvu de l'aiguille **(5)** dont l'extrémité distale **(6)** est tournée en direction opposée de la chambre de pressurisation **(8)** tandis que l'extrémité proximale **(7)** s'étend à l'intérieur de la chambre de pressurisation **(8)** selon une direction parallèle à la direction de rapprochement et/ou d'écartement.

17. Dispositif selon la revendication 16, **caractérisé en ce que** le piston **(11)** est pourvu d'un manchon de protection (**16**) entourant l'extrémité distale **(6)** de l'aiguille **(5),** en délimitant un volume de réception pour au moins une partie du récipient **(3).**

18. Dispositif selon la revendication 17, **caractérisé en ce que** le corps **(9)** de réception du piston **(11)** est pourvu au niveau de son fond situé dans le prolongement du piston, du septum (**20**) pénétrable par l'extrémité proximale **(7)** de l'aiguille.

19. Dispositif selon l'une des revendications 15 à 18, **caractérisé en ce que** la chambre **(24)** de distribution du liquide est délimitée par une partie du corps de réception (9) du piston (11) ou par un boitier muni du système filtrant (30) et rapporté sur le corps (9).

20. Dispositif selon l'une des revendications 12 à 19, **caractérisé en ce qu'**il comporte un système **(18)** de réglage de la pression de la chambre de pressurisation **(8)** en fonction du volume de fluide contenu à l'intérieur du récipient (**3**).

21. Dispositif selon l'une des revendications 12 à 20, **caractérisé en ce qu'**il comporte une butée de limitation du mouvement de rapprochement dans une position prédéterminée dans laquelle l'aiguille **(5)** traverse, par son extrémité proximale **(7),** le septum (**20**) pour déboucher dans la chambre **(24)** de distribution du liquide.

## Patentansprüche

1. Verfahren zum Übertragen eines Teils einer Flüssigkeit, die in einem Behälter (3) enthalten ist, der mit einem Stopfen (4) versehen ist, das darin besteht:
- mittels einer Hohlnadel (5), die durch den Stopfen (4) des Behälters hindurchgeht, das Innere des Behälters und eine dichte Druckbeaufschlagungskammer (8) einer Flüssigkeit, die mit einem Septum (20) ausgestattet ist, das durch eine Nadel durchdringbar ist und ein variables Volumen infolge der Relativbewegung zwischen der Hohlnadel (5) und dem Septum (20), das durch eine Nadel durchdringbar ist, aufweist, in Verbindung zu bringen,
- die relative Annäherung zwischen der Hohlnadel (5) und dem Septum (20), das durch eine Nadel durchdringbar ist, gemäß eines bestimmten Hubs sicherzustellen, um den Druck im Inneren der Kammer (8) und folglich des Behälters durch das Übertragen der Flüssigkeit durch die Hohlnadel zu erhöhen und einen Übertragungsdrucks unmittelbar vor dem Durchtreten der Hohlnadel durch das Septum (20) zu erreichen,
- die relative Annäherung zwischen der Hohlnadel (5) und dem Septum (20), das durch eine Nadel durchdringbar ist, fortzusetzen, um in eine Verteilungskammer (24) der Flüssigkeit zu münden, die einen Druck aufweist, der niedriger als der Übertragungsdruck ist, um unter der Einwirkung dieses Druckunterschieds das Übertragen eines Teils der Flüssigkeit des Behälters (3) durch die Hohlnadel (5) in die Verteilungskammer (24) sicherzustellen,
**dadurch gekennzeichnet, dass** es darin besteht, das Fließen der Flüssigkeit, die in die Verteilungskammer (24) übertragenen wird, durch ein Filtersystem (30) zu gewährleisten, um das Extrahieren eines Teils der Flüssigkeit zu gewährleisten.

2. Verfahren nach Anspruch 1, **dadurch gekennzeichnet, dass** es darin besteht, für einen Behälter (3), der Blut enthält, das Extrahieren des Plasmas als Teil der Flüssigkeit zu gewährleisten.

3. Verfahren nach Anspruch 2, **dadurch gekennzeichnet, dass** es darin besteht, nach dem Extrahieren des Plasmas das Filtersystem (30) für Analysen wiederherzustellen.

4. Verfahren nach einem der Ansprüche 1 bis 3, **dadurch gekennzeichnet, dass** die Flüssigkeit der Druckbeaufschlagungskammer (8) ein Behandlungsmedium für die Flüssigkeit, die in dem Behälter (3) enthalten ist, und eine ausreichende Menge an Gas aufweist, um das Komprimieren/die Druckbeaufschlagung zu genehmigen.

5. Verfahren nach Anspruch 2, **dadurch gekennzeichnet, dass** es darin besteht, einen Übertragungsdruck zu erreichen, der zwischen dem atmosphärischen Druck und dem atmosphärischen Druck plus 3 bar liegt, um das Extrahieren des Plasmas zu gewährleisten.

6. Verfahren nach einem der Ansprüche 1 bis 5, **dadurch gekennzeichnet, dass** es darin besteht, am Ende des Fließens der Flüssigkeit in die Verteilungskammer (24), das relative Beabstanden zwischen der Hohlnadel (5) und dem Septum (20), das durch eine Nadel durchdringbar ist, derart zu gewährleisten, dass die Hohlnadel (5) mit dem Inneren der Druckbeaufschlagungskammer (8) verbunden wird, um durch die unter Druck stehende Flüssigkeit der Kammer die Rückkehr der Flüssigkeit, die in der Nadel (5) enthalten ist, ins Innere des Behälters (3) zu gewährleisten.

7. Verfahren nach einem der Ansprüche 1 bis 6, **dadurch gekennzeichnet, dass** es darin besteht, den Druck der Druckbeaufschlagungskammer (8) in Abhängigkeit von dem Flüssigkeitsvolumen, das im Inneren des Behälters (3) enthalten ist, anzupassen.

8. Verfahren nach Anspruch 7, **dadurch gekennzeichnet, dass** es darin besteht, den Druck der Druckbeaufschlagungskammer (8) einzustellen, indem das unter atmosphärischen Druck Setzen der Druckbeaufschlagungskammer gewährleistet wird, solange der Abstand zwischen der Hohlnadel (5) und dem Septum (20), das durch eine Nadel durchdringbar ist, keinen bestimmten Wert erreicht.

9. Verfahren nach einem der Ansprüche 1 bis 8, **dadurch gekennzeichnet, dass** es darin besteht, das innere des Behälters (3) und die Druckbeaufschlagungskammer (8) in Verbindung zu setzen, indem der Behälter dahin gebracht wird, dass die Hohlnadel (5), die von der Druckbeaufschlagungskammer getragen wird und in diese Letztere mündet, den Stopfen (4) des Behälters durchquert.

10. Verfahren nach einem der Ansprüche 1 bis 9, **dadurch gekennzeichnet, dass** es darin besteht, bei einer Kalibrierungsphase vor dem in Verbindung Setzen des Behälters (3) mit der Druckbeaufschlagungskammer (8) das Septum (20) und die Hohlnadel (5) in einer Näheposition anzuordnen und durch die Hohlnadel (5) eine Druckflüssigkeit in die Kammer (8) einzuspritzen.

11. Verfahren nach Anspruch 1, **dadurch gekennzeichnet, dass** das System der relativen Annäherung (22) am Ende des Fließens der Flüssigkeit in die Verteilungskammer (24) gesteuert wird, um ein relatives Beabstanden zwischen der Hohlnadel (5) und dem Septum (20), das durch eine Nadel durchdringbar ist, derart zu gewährleisten, um die Hohlnadel aus dem Septum (20) zu entnehmen, damit diese Letztere mit dem Inneren der Druckbeaufschlagungskammer (8) verbunden wird, um durch die unter Druck stehende Flüssigkeit die Rückkehr der Flüssigkeit, die in der Nadel (5) enthalten ist, ins Innere des Behälters (3) zu gewährleisten.

12. Vorrichtung zum Übertragen eines Teils einer Flüssigkeit, die in einem Behälter (3) enthalten ist, der mit einem Stopfen (4) versehen ist, **dadurch gekennzeichnet, dass** sie aufweist:
- eine Hohlnadel (5), die mit einem distalen Ende (6) zum Eindringen durch den Stopfen des Behälters und einem proximalen Ende (7) versehen ist,
- eine Druckbeaufschlagungskammer (8) einer Flüssigkeit, durch die das proximale Ende (7) der Hohlnadel zur Übertragung hindurchgeht und die mit einem Septum (20) ausgestattet ist, das angeordnet ist, um durch das proximale Ende (7) der Nadel nach deren Bewegung durchdringbar zu sein, wobei diese Kammer (8) dicht ist und durch das Septum (20) von einer Verteilungskammer (24) der Flüssigkeit getrennt ist, in die das proximale Ende der Hohlnadel (5) münden kann, und
- ein System der relativen Annäherung (22) zwischen der Hohlnadel (5) und dem Septum (20), um eine Erhöhung des Drucks der Flüssigkeit infolge der Annäherung zu gewährleisten, wobei dieses System der Annäherung gemäß eines bestimmten Hubs gesteuert ist, um in einer ersten Phase den Druck im Inneren der dichten Kammer (8) zu erhöhen, bis ein Übertragungsdruck unmittelbar vor dem Durchtreten der Hohlnadel (5) durch das Septum (20) erreicht ist, und in einer zweiten Phase, dass die Hohlnadel (5) das Septum (20) durchquert, um in eine Verteilungskammer (24) der Flüssigkeit zu münden, die einen Druck aufweist, der niedriger als der Übertragungsdruck ist, um unter der Einwirkung dieses Druckunterschieds das Übertragen eines Teils der Flüssigkeit des Behälters (3) in die Verteilungskammer (24) sicherzustellen, wobei die Verteilungskammer (24) ein Filtersystem (30) aufweist, um das Extrahieren einer flüssigen Phase aus der Flüssigkeit, die in die Kammer übertragen ist, zu gewährleisten.

13. Vorrichtung nach Anspruch 12, **dadurch gekennzeichnet, dass** das Filtersystem (30) ein Filtermedium (31) aufweist, das sich in einem Abstand von dem Septum, das durch eine Nadel durchdringbar ist, erstreckt, um die Verteilung der Flüssigkeit auf dem Filtermedium (31) zu gewährleisten.

14. Vorrichtung nach Anspruch 13, **dadurch gekennzeichnet, dass** das Filtermedium (31) auf einer Trägerstruktur in Position gehalten wird, auf der das Filtermedium ruht.

15. Vorrichtung nach einem der Ansprüche 12 bis 14, **dadurch gekennzeichnet, dass** das System der Annäherung (22) einen Aufnahmekörper (9) für einen Kolben (11) aufweist, die zwischen sich die Druckbeaufschlagungskammer (8) begrenzen, wobei der Aufnahmekörper (9) mit dem Septum (20) ausgestattet ist und der Kolben (11) und der Körper relativ zueinander unter der Einwirkung einer mechanischen Schubbeanspruchung translatorisch beweglich sind, die entweder auf die Hohlnadel (5) in die Richtung f und/oder auf das Septum (20) in die Richtung f1 oder auf die Hohlnadel (5) in die Richtung f1 und/oder auf das Septum (20) in die Richtung f ausgeübt wird.

16. Vorrichtung nach Anspruch 15, **dadurch gekennzeichnet, dass** der Kolben (11) mit der Nadel (5) versehen ist, deren distales Ende (6) in die entgegengesetzte Richtung der Druckbeaufschlagungskammer (8) gerichtet ist, während sich das proximale Ende (7) im Inneren der Druckbeaufschlagungskammer (8) in einer Richtung erstreckt, die parallel zu der Richtung der Annäherung und/oder Beabstandung ist.

17. Vorrichtung nach Anspruch 16, **dadurch gekennzeichnet, dass** der Kolben (11) mit einer Schutzmanschette (16) versehen ist, die das distale Ende (6) der Nadel (5) umgibt, wodurch ein Aufnahmevolumen für mindestens einen Teil des Behälters (3) definiert wird.

18. Vorrichtung nach Anspruch 17, **dadurch gekennzeichnet, dass** der Körper (9) zum Aufnehmen des Kolbens (11) an seinem Boden, der sich in der Verlängerung des Kolbens befindet, mit dem Septum (20), das durch das proximale Ende (7) der Nadel durchdringbar ist, versehen ist.

19. Vorrichtung nach einem der Ansprüche 15 bis 18, **dadurch gekennzeichnet, dass** die Verteilungskammer (24) der Flüssigkeit durch einen Teil des Aufnahmekörpers (9) des Kolbens (11) oder durch ein Gehäuse begrenzt ist, das mit dem Filtersystem (30) versehen ist und an dem Körper (9) angebracht ist.

20. Vorrichtung nach einem der Ansprüche 12 bis 19, **dadurch gekennzeichnet, dass** es ein System (18) zum Einstellen des Drucks der Druckbeaufschlagungskammer (8) in Abhängigkeit von dem Flüssigkeitsvolumen, das im Inneren des Behälters (3) enthalten ist, aufweist.

21. Vorrichtung nach einem der Ansprüche 12 bis 20, **dadurch gekennzeichnet, dass** es einen Anschlag zur Begrenzung der Annäherungsbewegung in einer vorbestimmten Position aufweist, in der die Nadel (5) durch ihr proximales Ende (7) das Septum (20) durchquert, um in die Verteilungskammer (24) der Flüssigkeit zu münden.

## Claims

1. A method of transferring a portion of a liquid contained in a container (3) provided with a plug (4), consisting:
• using a hollow needle (5) passing through the plug (4) of the container, in putting the inside of the container into communication with a leaktight chamber (8) for pressurizing a fluid, which chamber is provided with a septum (20) that is penetrable by needle and presents a volume that is variable as a result of relative movement between the hollow needle (5) and the septum (20) that is penetrable by needle;
• in causing the hollow needle (5) and the septum (20) that is penetrable by needle to approach each other over a determined stroke so as to increase the pressure inside the chamber (8) and consequently inside the container by transferring the fluid via the hollow needle and reach a transfer pressure immediately prior to the hollow needle passing through the septum (20); and
• in continuing to cause the hollow needle (5) and the septum (20) that is penetrable by needle to approach each other so that the hollow needle passes through the septum so as to open out into a liquid distribution chamber (24) at a pressure that is lower than the transfer pressure so that under the effect of this pressure difference a portion of the liquid is transferred through the hollow needle (5) from the container (3) into said distribution chamber (24),
**characterized in that** it consists in ensuring that the liquid transferred into the distribution chamber (24) flows through a filter system (30) in order to extract a portion of the liquid.

2. The method according to claim 1, **characterized in that** it consists, for a container (3) containing blood, in ensuring that the portion of the liquid that is extracted is plasma.

3. The method according to claim 2, **characterized in that** it consists, after plasma has been extracted, in recovering the filter system (30) for analysis purposes.

4. The method according to any one of claims 1 to 3, **characterized in that** the fluid in the pressure chamber (8) comprises a medium for processing the liquid contained in the container (3) and a quantity of gas that is sufficient to enable compression/pressurization.

5. The method according to claim 2, **characterized in that** it consists, in order to ensure that plasma is extracted, in reaching a transfer pressure that lies in the range atmospheric pressure to atmospheric pressure plus 3 bars.

6. The method according to any one of claims 1 to 5, **characterized in that** it consists, at the end of the fluid flowing into the distribution chamber (24), in enabling the hollow needle (5) and the septum (20) that is penetrable by needle to be separated relative to each other in such a manner that the hollow needle (5) communicates with the inside of the pressure chamber (8) so as to enable the fluid under pressure in said chamber to cause the liquid contained in the needle (5) to be returned into the container (3).

7. The method according to any one of claims 1 to 6, **characterized in that** it consists in adapting the pressure in the pressure chamber (8) as a function of the volume of fluid contained inside the container (3).

8. The method according to claim 7, **characterized in that** it consists in adjusting the pressure in the pressure chamber (8) by ensuring that the pressure chamber is set to atmospheric pressure so long as the spacing between the hollow needle (5) and the septum (20) that is penetrable by needle does not reach a determined value.

9. The method according to any one of claims 1 to 8, **characterized in that** it consists in putting the inside of the container (3) into communication with the pressure chamber (8), by guiding the container so that the hollow needle (5) carried by the pressure chamber and opening out therein, passes through the plug (4) of the container.

10. The method according to any one of claims 1 to 9, **characterized in that** it consists, during a calibration stage prior to putting the container (3) into communication with the pressure chamber (8), in placing the septum (20) and the hollow needle (5) in a position of proximity and in injecting a pressurizing fluid into the chamber (8) through the hollow needle (5).

11. The method according to claim 1, **characterized in that** the relative approach system (22) is controlled at the end of the fluid flowing into the distribution chamber, for enabling the hollow needle (5) and the septum (20) that is penetrable by needle to be separated relative to each other, so as to extract the hollow needle from the septum (20) in such a manner that this hollow needle communicates with the inside of the pressure chamber (8) so as to enable the fluid under pressure to cause the liquid contained in the needle (5) to be returned into the container (3).

12. A device for transferring a portion of a liquid contained in a container (3) provided with a plug (4), **characterized in that** it comprises:
• a hollow needle (5) provided with a proximal end (7) and with a distal end (6) for penetrating through the plug of the container;
• a chamber (8) for pressurizing a fluid and having the proximal end (7) of the hollow transfer needle passing therethrough, the chamber being provided with a septum (20) positioned to be penetrable by the proximal end (7) of the needle after the needle has moved, this chamber (8) being leaktight and separated by means of the septum (20) from a liquid distribution chamber (24) into which the proximal end of the hollow needle (5) can open out; and
• a system (22) for causing the hollow needle (5) and the septum (20) to approach each other so as to increase the pressure of the fluid as a result of said approach, this approach system being controlled to move over a determined stroke so that in a first stage the pressure inside the leaktight chamber (8) is increased up to a transfer pressure immediately prior to the hollow needle (5) passing through the septum (20), and in a second stage the hollow needle (5) passes through the septum (20) to open out into the liquid distribution chamber (24) that is at a pressure lower than the transfer pressure so that under the effect of this pressure difference a portion of the liquid is transferred from the container (3) into said distribution chamber (24), the distribution chamber (24) including a filter system (30) for ensuring that a liquid phase is extracted from the liquid transferred into said chamber.

13. The device according to claim 12, **characterized in that** the filter system (30) includes a filter medium (31) that lies at a distance from the septum that is penetrable by needle so as to ensure that the liquid is distributed over the filter medium (31).

14. The device according to claim 13, **characterized in that** the filter medium (31) is held in position on a support structure on which the filter medium rests.

15. The device according to any one of claims 12 to 14, **characterized in that** the approach system (22) comprises a cylinder (9) for receiving a piston (11) together defining the pressure chamber (8), the receiving cylinder (9) being provided with the septum (20) and the piston (11) and the cylinder being movable in translation relative to each other under the action of a mechanical thrust force exerted either on the hollow needle (5) in the direction f and/or on the septum (20) in the direction f1, or on the hollow needle (5) in the direction f1 and/or on the septum (20) in the direction f.

16. The device according to claim 15, **characterized in that** the piston (11) is provided with the needle (5) having its distal end (6) pointing away from the pressure chamber (8) and its proximal end (7) extending inside the pressure chamber (8) in a direction parallel to the approach and/or separation direction.

17. The device according to claim 16, **characterized in that** the piston (11) is provided with a protective sleeve (16) surrounding the distal end (6) of the needle (5), by defining a volume for receiving at least a portion of the container (3).

18. The device according to claim 17, **characterized in that** the cylinder (9) for receiving the piston (11) is provided, at its end wall situated in line with the piston, with the septum (20) that can be penetrated by the proximal end (7) of the needle.

19. The device according to any one of claims 15 to 18, **characterized in that** the liquid distribution chamber (24) is defined by a portion of the cylinder (9) for receiving the piston (11) or by a separate unit containing the filter system (30) and fitted to the cylinder (9).

20. The device according to any one of claims 12 to 19, **characterized in that** it includes a system (18) for adjusting the pressure in the pressure chamber (8) as a function of the volume of fluid contained inside the container (3).

21. The device according to any one of claims 12 to 20, **characterized in that** it includes an abutment for limiting the approach movement in a predetermined position in which the proximal end (7) of the needle (5) passes through the septum (20) to open out into the liquid distribution chamber (24).
